# EUROPEAN PATENT APPLICATION

(11) **EP 4 019 014 A1**
(43) Date of publication of application: **29.06.2022**
(21) Application number: 20217100.5
(22) Date of filing: 23.12.2020
(51) Int. Cl.: A61K 31/05, A61K 9/00, A61P 17/00, A61K 36/00

(54) **CANNABIDIOL COMPOSITIONS AND USES THEREOF**

(71) Applicant: Pharmotech SA, 1228 Plan les Ouates (CH)
(72) Inventor: AESCHBACH, Rodin, 1217 Meyrin (CH)
(74) Representative: reuteler & cie SA

(57) **Abstract**

The present invention is directed to use of cannabidiol and compositions thereof as preventive and curative agent for IBH. The invention is further directed to related methods and compositions.

## Description

### Field of the Invention

The present invention relates to the field of equine insect bite hypersensitivity (IBH). In particular, the invention relates to the use of cannabinoid compositions as preventive and curative agent for IBH.

### Background of the Invention

Hypersensitivity is an excessive immune response to foreign bodies, leading to inflammation and organ dysfunction. Both hypersensitivity and autoimmunity are mediated by similar inflammatory pathways, but they are distinguished by the source of the triggering antigen: hypersensitivity is directed against foreign antigens, while autoimmunity is directed against self-antigens that are misread as non-self-antigens by the immune system. Allergies include immune responses to antigens, leading to protective (immune) and adverse (hypersensitivity) reactions (Swiderski, 2000, Vet Clin North Am Equine Pract, 16(1), 131-51, vii).

Hypersensitivity can be roughly divided into two categories: antibody-mediated reactions and T lymphocyte-mediated reactions. The antibody-mediated response is immediate because the inflammatory pathway and subsequent pathology begin when the antigen and antibody bind, although these events may be delayed during antibody synthesis if the subject has not previously been sensitized. In contrast, during the recruitment of effector cells, the pathological results of the T lymphocyte-mediated response of sensitized individuals are delayed by 1 to 3 days (*Swiderski, 2000, supra*).

The Gell and Coombs classification of hypersensitivity is the most widely used, and distinguishes four types of immune response which result in bystander tissue damage. Type I hypersensitivity is not associated with autoimmune disease. It is produced by IgE-mediated mast cell degranulation. Type II hypersensitivity is responsible of tissue and organ injury mediated by specific antibodies. More specifically, it is produced by the cell killing due to binding of cytotoxic IgG or IgM antibodies to antigens on cell surface. Type III hypersensitivity results from deposition of immune complexes, which initiates activation of the classical complement cascade, as well as recruitment and activation of phagocytes and CD4+ lymphocytes. The site of immune complex deposition is determined by the relative amount of antibody, size of the immune complexes, nature of the antigen and local hemodynamics. Finally, type IV hypersensitivity is mediated by activated T cells and macrophages, which together cause tissue damage *(*Ralston, et al., 2018, Davidson's Principles and Practice of Medicine, IE Edition, 23rd Ed. 2018, Elsevier*).*

Equine hypersensitivity remains untapped and relies on findings in human and rodent models. Equine insect bite hypersensitivity (IBH) also called Queensland Itch, sweet itch or summer eczema (SE) or Kasen or *Culicoides* hypersensitivity (Schaffartzik et al., 2012, Vet Immunol Immunopathol., 2012;147:113-126*)* is a chronic relapsing seasonal episodes of itching felt by affected horses cause them to scratch and traumatize their manes, tails and chests down to bleeding tissue which is caused by the biting of insects of the genus *Culicoides.* The *Culicoides* inject salivary proteins as they feed that can elicit Type 1 and 4 allergic responses in predisposed horses. *Culicoides* species midges are found in various areas of the world (Fadok et al., 1990, Equine Vet J, 22, 236-240*;* Greiner et al., 1990, Med Vet Entomol., 375-381*).*

Hairless, weeping, and sometimes even ulcerative lesions caused by inflammation and severe itching are typical symptoms of this seasonal and refractory chronic disease. Lesions are characterized by hyperkeratosis, lichenification of the skin, bleeding, swelling, scales, and crust formation. Histologic hallmarks of IBH lesions are thickening of the stratum corneum, epidermis, and dermis, with abundant fibrosis in the latter (*Schaffartzik et al., 2012, supra;* Antonia Fettelschoss-Gabriel et al., 2018, Journal of Allergy and Clinical Immunology, 142 (4), 1194-1205*).* On a cellular level, the skin lesions are characterized by massive eosinophil infiltration caused by an underlying allergic response.

IBH affected horses were also reported to react against other blood feeding insects like black flies (*Simulium* spp.), stable flies (*Stomoxys calcitrans*), mosquitoes and horseflies.

Although IBH was first described in 1840 and is currently the best characterized allergic disease in horses, treatment options are still poor, and currently, no satisfactory treatment of IBH is available. For example, corticosteroids are effective at reducing the effects of itch but have unwanted side effects and do not treat the cause of the disorder. Therapeutic vaccines against equine IL-5 (eIL-5) and IL-31 have been developed for symptomatic treatment of IBH (*Antonia Fettelschoss-Gabriel et al., 2018, supra;* Jonsdottir et al., 2019, Curr Derm Rep, 8, 303-312) and Allergen-specific immunotherapy (ASIT) has been investigated on the basis of the availability of a large panel of pure recombinant Culicoides allergens relevant for IBH (*Jonsdottir et al., 2019, supra).*

Due to the severity of this disorder, which, if not treated would commonly favour secondary opportunistic infections with bacteria, mites, and fungi which can cause further local irritation, enhancing lesion formation, it would be highly desirable to have new methods for preventing and/or treating equine IBH.

### Summary of the invention

The present invention relates to the unexpected finding that cannabidiol (CBD) is active in the prevention and treatment of insect bite hypersensitivity, in particular in horses. In particular, CBD was able to reduce pruritus score when applied topically to the IBH skin lesions.

An aspect of the invention provides CBD or a composition thereof for use in the prevention and treatment of insect bite hypersensitivity (IBH), in particular in horses.

An aspect of the invention provides a use of CBD or a composition thereof for the preparation of a pharmaceutical preparation for the prevention and/or treatment of IBH.

Another aspect of the invention relates to a veterinary composition comprising CBD at a concentration between about 0.001 µg/mL to 1'000 mg/mL, for example from about 0,001µg/mL to 1000mg/mL, and a further veterinary acceptable carrier, diluent or excipient.

Another aspect of the invention relates to a method for preventing and/or treating IBH in a subject (horses), said method comprising administering a therapeutically effective amount of CBD, or a composition thereof to a subject in need thereof.

### Description of the figures

**Figure 1** represents the effects of CBD formulation according to the invention on IBH horse skin lesions as described in Example 1. A: Puritus score versus time after start of the treatment;
**B:** Pruritus observation for each horse over the treatment time.
**Figure 2** represents the effects of CBD formulation according to the invention on IBH horse skin lesions as described in Example 1. A: IBH score versus time after start of the treatment;
**B:** Pruritus observation for each horse over the treatment time.
**Figure 3** shows photos of the IBH lesions before (A) and 28 days after the treat of the treatment **(B).**

### Detailed description of the invention

The term "insect bite hypersensitivity (IBH)" refers to a disorder as described herein. It is an IgE-mediated dermatitis caused by bites of *Culicoides* spp., which occurs frequently in horses imported from Iceland to continental Europe. Classical diagnosis of IBH combines the history of the horse and the observation of clinical signs that follow a seasonal pattern (Jose Paes Oliveira-Filho et al., 2012, Ciências Agrárias, Londrina, v. 33, n. 3, p. 1113-1122). These methods may fail to detect some affected horses and a diagnostic test that could give a more reliable observation of the horse's sensitivity to Culicoides spp. as described in Van der Meide et al., 2014, Vet J, 200(1):31-7*.*

As used herein, "treatment" and "treating" and the like generally mean obtaining a desired pharmacological and physiological effect. The effect may be prophylactic in terms of preventing or partially preventing the disease, symptom or condition thereof and/or may be therapeutic in terms of a partial or complete cure of the inflammation and hypersensitivity symptoms. The term "efficacy" of a treatment or method according to the invention can be measured based on changes in the course of disease or condition in response to a use of a compound or a method according to the invention. For example, the efficacy of a treatment or method according to the invention can be measured by its impact on signs or symptoms of inflammation and hypersensitivity. A response is achieved when the subject experiences partial or total alleviation, or reduction of unwanted symptoms.

According to one aspect, the efficacy of a treatment according to the invention can be assessed by the effect of an effective amount of CBD on the pruritus, inflammation and hair loss score. The term "effective amount" as used herein refers to an amount of CBD, or a formulation thereof that elicits a detectable reduction of the symptoms of the disease in a subject that is being administered said compound or formulation.

### Compounds according to the invention

The term "cannabidiol (CBD)" refers to a type of cannabinoid that can be found in cannabis plant having the following chemical structure: 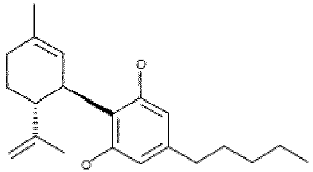 2-[(1R,6R)-6-isopropenyl-3-methylcyclohex-2-en-1-yl]-5-pentylbenzene-1,3-diol, also named s Δ²-cannabidiol.

It is a major constituent of the Cannabis plant, second to THC, and represents up to 40% in its extracts. Compared with THC has a very low affinity for CB1 and CB2 receptors which results in this substance being non-psychoactive. CBD can be extracted from various Cannabis plant species including *Cannabis sativa, indica* and *ruderalis.* In particular, CBD can be extracted as a pure compound from genetically modified cannabis plant which is producing increased levels of CBD as compared to naturally occurring plants.

According to one embodiment, is provided a CBD of a natural origin, that is extracted from *Cannabis* strains variety.

According to another embodiment, a CBD can be isolated by standard methods known to the skilled person, for example comprising collecting of plant material and extraction and purification.

Alternatively, CBD may be prepared by synthetic methods.

### Methods and uses according to the invention

According to a particular embodiment, are provided CBD or a composition thereof for use in the prevention and/or treatment of insect bite hypersensitivity, in particular in horses.

Those skilled in the treatment of summer itch will easily determine the preventive or therapeutically effective amount of CBD from the test results presented hereinafter. In general, it is contemplated that an effective dose will be from about 0,001mg/kg to about 1000mg/kg of body weight, more preferably from about 0,001mg/kg to about 1000mg/kg of body weight of the warm-blooded animal to be treated. It may be appropriate to administer the therapeutically effective dose in the form of two or more sub-doses at appropriate intervals throughout the day.

### Compositions according to the invention

Compositions or formulations according to the invention may be administered as a pharmaceutical formulation.

Pharmaceutical compositions of this invention may further comprise one or more pharmaceutically acceptable additional ingredient(s) such as alum, stabilizers, antimicrobial agents, buffers, coloring agents, flavoring agents, adjuvants, and the like.

Compositions of the invention and unit dosages thereof, and in such form may be employed as liquids such as solutions, suspensions, emulsions, elixirs. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Compositions of this invention may also be liquid formulations including, but not limited to, aqueous or oily suspensions, solutions and emulsions.

Liquid forms suitable for topical administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives including, but not limited to, veterinary acceptable aqueous solvents such as ethanol, suspending agents, emulsifying agents, non-aqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, vegetal oil such as almond oil or sesame oil, cannabis oil, fractionated coconut oil, oily esters, propylene glycol, and ethyl alcohol. Preservatives include, but are not limited to, methyl or propyl p-hydroxybenzoate and sorbic acid. Compositions of this invention may also be formulated as a liposome preparation. The liposome preparation can comprise liposomes which penetrate the cells of interest or the *stratum corneum*, and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the *stratum corneum.*

The compounds of this invention can also be administered in sustained release forms or from sustained release drug delivery systems. A description of representative sustained release materials can also be found in the incorporated materials in *Remington's Pharmaceutical Sciences.* For topical application dip, spray, powder, dust, pour- on, spot-on, emulsifiable concentrate, jetting fluid, shampoos, creams, ointments, collar, patches, tag or harness may be used. Such formulations are prepared in a conventional manner in accordance with standard veterinary and pharmaceutical practice.

According to a particular embodiment, compositions of the invention are veterinary compositions.

Further materials as well as formulation processing techniques and the like are set out in *The* Science and Practice of Pharmacy, 23rd Edition, 2020, Adeboye Adejare, Academic Press which is incorporated herein by reference.

According to a particular aspect, a CBD composition according to the invention contains from about 0.5 to 1% (weight (w)/weight (w)) CBD.

According to another particular aspect, a CBD composition according to the invention is an oil or an aqueous formulation.

According to another further particular aspect, an aqueous CBD composition according to the invention contains from about 1 to 80 % (w/w) ethanol (e.g. 70%w/w).

According to another further particular aspect, a CBD composition according to the invention contains from about 5 to 25 % (w/w) propylene glycol (e.g. 20% w/w).

According to another further particular aspect, an aqueous CBD composition according to the invention contains from about 1 to 90 % (w/w) water (e.g. 9.5% w/w).

According to another further particular aspect, an aqueous CBD composition according to the invention contains CBD 0.5% (w/w), ethanol 70%, propylene glycol 20% and water 9.5%.

### Mode of administration

Compositions of this invention may be administered in any manner including, but not limited to topically administration.

Compositions of this invention may also be administered topically to the skin, in particular locally for example by a local spray of a formulation according to the invention. Compositions according to the invention may be administered to a subject in need thereof as a single or as a repeated administration.

Compositions of the invention may be in a dosage unit form. "Dosage unit form" as used herein refers to physically discrete units suitable as unitary dosages, each unit containing a predetermined amount of active ingredient calculated to produce the desired therapeutic effect in association with the required pharmaceutical carrier. Examples of such dosage unit forms are patches and the like, and segregated multiples thereof.

Compositions according to this invention may be administered to a subject in need thereof prior to, simultaneously or sequentially with other therapeutic regimens.

According to a particular aspect, a CBD composition containing about 0.5% CBD is applied twice daily.

The dosage administered, as single or multiple doses, to an individual will vary depending upon a variety of factors, including pharmacokinetic properties, subject conditions and characteristics (sex, age, body weight, health, size), extent of symptoms, concurrent treatments, frequency of treatment and the effect desired.

### Subjects

In an embodiment, subjects according to the invention are mammals, in particular horses suffering or at risk of suffering from IBH.

References cited herein are hereby incorporated by reference in their entirety. The present invention is not to be limited in scope by the specific embodiments and drawings described herein, which are intended as single illustrations of individual aspects of the invention, and functionally equivalent methods and components are within the scope of the invention.

### EXAMPLES

The following abbreviations refer respectively to the definitions below:
**CBD** (cannabidiol).

### Example 1: Use of a topical formulation of CBD in horses diagnosed with IBH

An oil formulation of CBD containing 1% (w/w) CBD in sesame oil was prepared and applied once daily on the skin's lesions of 11 animals (*Equus caballus* (horse) and *Equus ferus caballus* (poney)) with a confirmed diagnosis of IBH with mild to moderate stage over 4 weeks period and protecting the lesions with a blanked to protect the lesions from sunlight and further insect bites. The application was made by hand (using a cotton) and a glove was used for massaging the product into the affected skin area. Each horse received at minimum 1 bottle of 100 ml on Day 1 and 1 Bottle on Day 14, administration was indicated to be daily. Larger volumes have been used for larger lesions.

Safety parameters were measured by hematology measures and serum amyloid A (SSA) measures and IBH lesion score was calculated as detailed in Fettelschoss-Gabriel et al., 2018, J Allergy Clin Immunol 142(4):1194-1205 at the beginning and at the end of the study as a disease specific observation. LC-MS/MS was used for CBD dosing to verify a limit of 0.50 mg/mL in whole blood. Pruritus was scored from 1-5 for each skin area affected and was calculated taking into account 3 main factors: tail scratching; mane scratching and belly scratching on ground/floor. SUM Score Pruritus - Day 1: 6 Pruritus score was calculated for Day 1, Day 14, Day 28

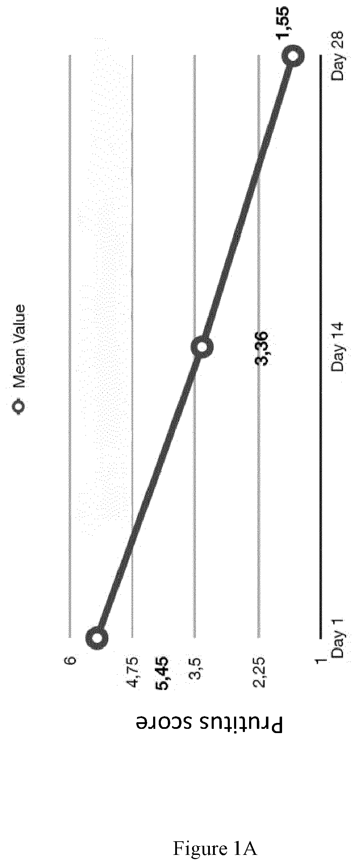

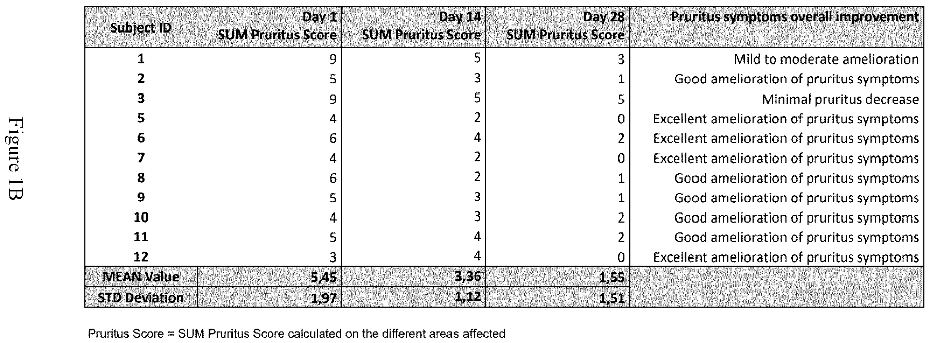

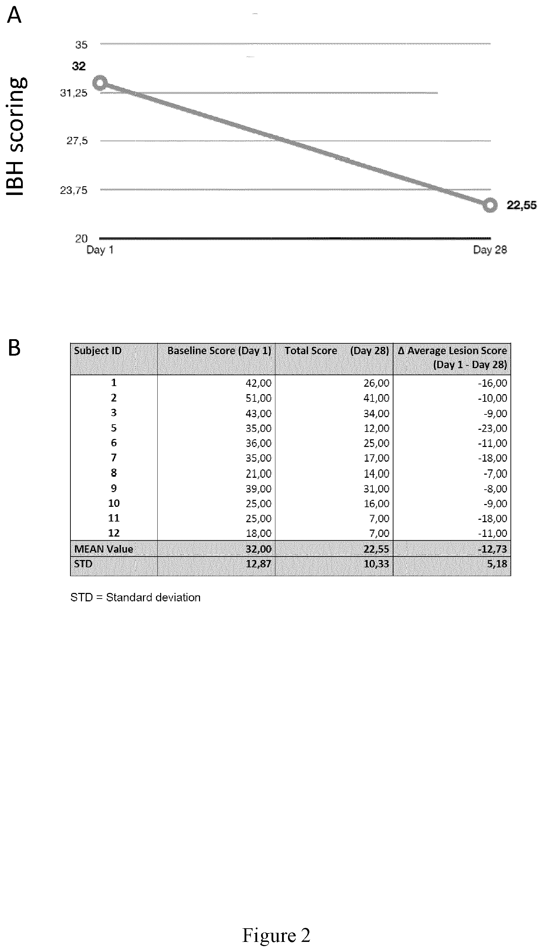

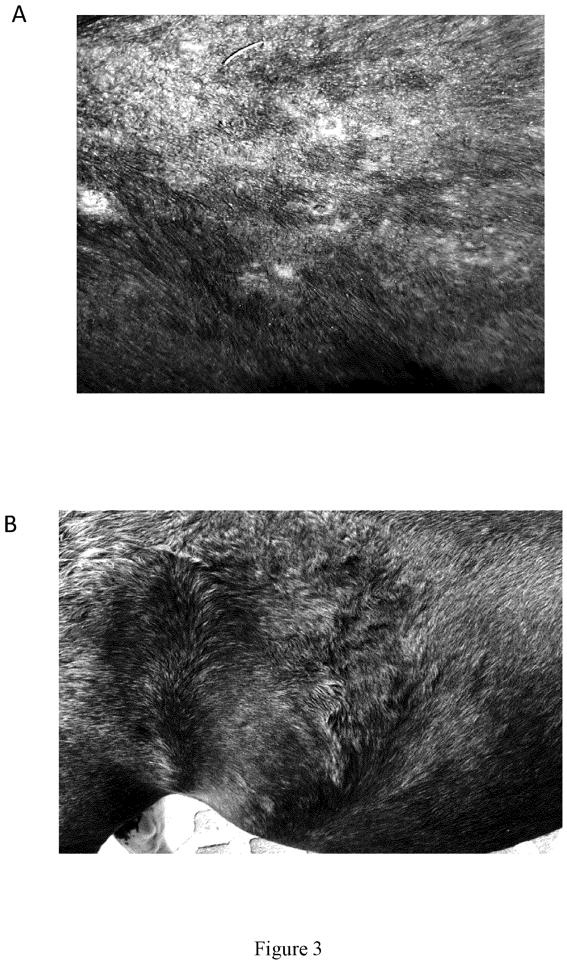

Then, the mean was calculated. Main pruritus signs are: tail scratching, mane scratching, face scratching and sometimes rolling on the ground for belly scratching. **Figure 1A** **and** **B** provide an overview on the mean pruritus score and **Figure 2A and B** an overview on the IBH score and their evolution during the CBD treatment. **Figure 3** shows a picture of IBH lesions before **(A)** and after **(B)** the 28 days of treatment.

As an overall result, a clear decrease of IBH and pruritus symptoms during treatment according to the invention was observed.

Moreover, CBD was not found in detectable concentrations in plasma which confirmed that at 1% topical concentration the systemic absorption is negligeable. No particular trend in eosinophile and WBC count was observed

## Claims

1. CBD or a composition thereof for use according to claim 1, wherein CBD or a composition thereof is to be administered topically on the skin lesions at first symptoms of IBH such as pruritus, inflammation and hair loss

2. CBD or a composition thereof for use according to claim 1, wherein CBD or a composition thereof is to be administered at a dose of 0.001% to about 10% (w/w).

3. A veterinary topical composition comprising CBD at a concentration between about 0.5 g to 1.0 g of CBD /per 100 g of formulated final product, CBD formulated in a veterinary acceptable carrier, diluent or excipient.

4. A veterinary composition according to claim 5, wherein said composition comprises about 0.5 to 1% (w/w) CBD.

5. A veterinary composition according to claim 5 or 6, wherein said composition is an oil or an aqueous formulation.

6. A veterinary composition according to any one of claim 5 to 7, further comprising from about 60 to 80 % ethanol (w/w).

7. A veterinary composition according to any one of claim 5 to 8, further comprising from about 10 to 25 % (w/w) propylene glycol.

8. A veterinary composition according to any one of claim 5 to 9 from about 9 to 10 % (w/w).

9. A veterinary composition according to any one of claim 5 to 8, further comprising from about 10 to 25 % (w/w) propylene glycol.

10. A veterinary composition according to any one of claim 5 to 9 from about 9 to 10 % (w/w) water.

11. A CBD composition for use according to any one of claims 1 to 4, wherein said composition is a veterinary composition according to any one of claims 5 to 10.

12. A method for preventing and/or treating insect bite hypersensitivity (IBH), said method comprising administering a therapeutically effective amount of CBD, or a composition thereof to a subject in need thereof.

13. A method according to claim 12, wherein CBD is to be administered topically on the skin lesions at first symptoms of IBH such as pruritus, inflammation and hair loss for example by spraying.

14. A method according to claim 13, wherein the CBD composition is a veterinary composition according to any one of claims 5 to 10.
